# EUROPEAN PATENT APPLICATION

(11) **EP 1 864 973 A1**
(43) Date of publication of application: **12.12.2007**
(21) Application number: 06011981.5
(22) Date of filing: 09.06.2006
(51) Int. Cl.: C07D 209/42

(54) **Process for the preparation of perindopril and salts thereof**

(71) Applicant: Sochinaz SA, 1895 Vionnaz (CH)
(72) Inventor: Haider, Akhtar, 1024 Eculens (CH); Megevand, Sophie, 1024 Ecublens (CH); Nicollier, Brigitte, 1920 Martigny (CH); Pannatier, Yvan, 1872 Trois Torrents (CH)
(74) Representative: Burtin, Jean-François

(57) **Abstract**

The present invention describes a novel method for the synthesis of (2S,3aS,7aS)-octahydroindole-2-carboxylic acid, or of one of its aryl esters of formula XIII 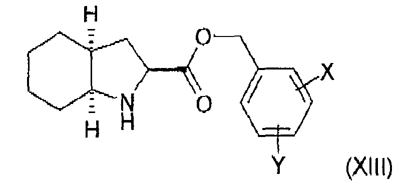 wherein X and Y are a hydrogen atom, a halogen, alkyl, alkoxyl or nitro group, and the conversion of the paranitrobenzyl ester of formula X 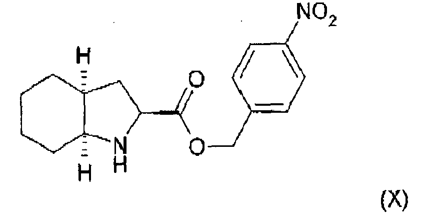 into Perindopril of formula I 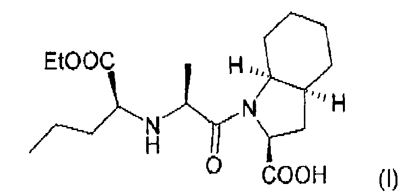 or one of its salts.

## Description

The present invention relates to chemistry and more particularly to organic synthesis.

This invention more precisely relates to a process for the industrial synthesis of Perindopril.

Perindopril and its salts, particularly the tert-butylamine salt (Perindopril erbumine) or the arginine salt, present useful pharmacological properties. They are especially known as valuable ACE inhibitors.

Perindopril is chemically defined as (2S,3aS,7aS)-1-{2-[1-ethoxycarbonyl-(S)-butylamino]-(S)-propionyl}-octahydroindole-2-carboxylic acid. Its chemical formula is pictured in formula I :

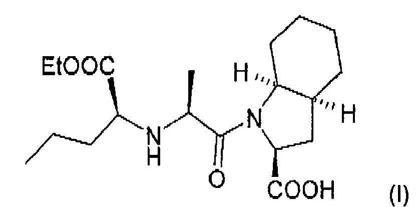

The first patent mentioning Perindopril is EP 0 049 658. No industrial synthesis of this compound is disclosed in this patent.

The first industrial scale synthesis of Perindopril is disclosed in EP 0 308 341. The key step of this synthesis is a peptide-coupling of N-[(S)-1-ethoxycarbonyl-butyl]-(S)-alanine with benzyl-(2S,3aS,7aS)-octahydroindole-2-carboxylate, using as a coupling agent dicyclohexylcarbodiimide in combination with 1-hydroxy benzotriazole. The benzyl ester is then debenzylated to give Perindopril of formula I, which may be converted into its tert-butylamine or arginine salt.

One of the main drawbacks of this process is that a significant amount (5-15%) of by-products of formula II and II' : 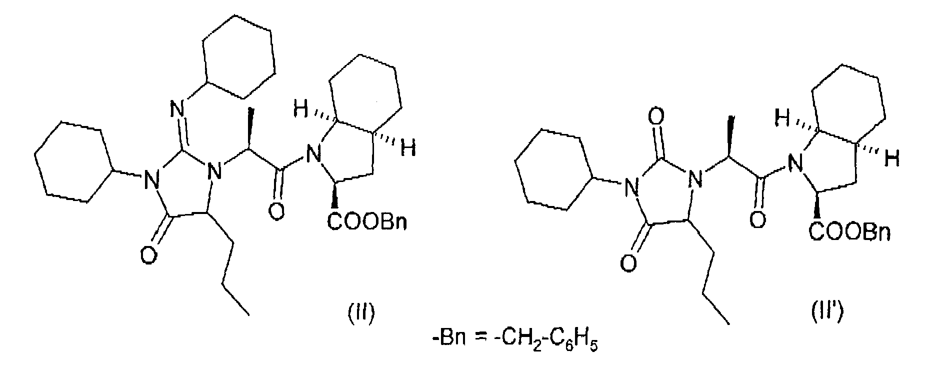 is formed during the coupling reaction. After debenzylation, Perindopril has to be submitted to several purification steps to reach a pharmaceutical purity level.

Many recent patents describe ways of avoiding these purification steps. The coupling reaction may be carried out by means of several coupling reagents different from the mixture dicyclohexylcarbodiimide/1-hydroxybenzotriazole. A wide choice of reagents is displayed in EP 1 420 029. Most of them are rather expensive, which is a drawback for an industrial scale synthesis.

It is also possible to use other intermediates than N-[(S)-1-ethoxycarbonyl-butyl]-(S)-alanine and benzyl-(2S,3aS,7aS)-octahydroindole-2-carboxylate. EP 1 333 026 discloses the use of the compound of formula III : 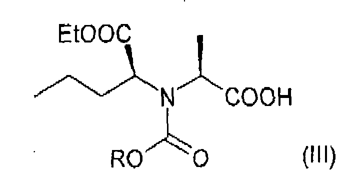 wherein R is a lower alkyl or a lower aralkyl group,
which may be converted into an activated derivative by thionyl chloride and then coupled with (2S,3aS,7aS)-octahydroindole-2-carboxylic acid in an inert organic solvent. The amine of compound (III) is deprotected during the coupling step and Perindopril erbumine is obtained by crystallization in the presence of tert-butylamine. Unfortunately, the yields are rather poor (35-55% of Perindopril erbumine), whereas the yield in EP 0 308 341 is above 87%.

Also EP 1 256 590 describes the coupling of the compounds of formulas (IV) and (V) : 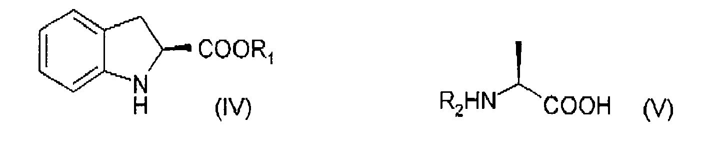 wherein R₁ is a hydrogen, an alkyl or a benzyl group and R₂ is a protective group,
in the presence of small amounts of dicyclohexylcarbodiimide and 1-hydroxybenzotriazole, which avoids the formation of unwanted by-products. This process needs more steps than the ones previously described to give access to Perindopril. Moreover, the last steps are not stereospecific.

Despite the formation of unwanted by-products, the process disclosed in EP 0 308 341 still presents many advantages, such as good yields, the formation of a single enantiomer as the product and a low number of steps, using cheap and easily available coupling reagents. Moreover, the starting materials N-[(S)-1-ethoxycarbonyl-butyl]-(S)-alanine and (2S,3aS,7aS)-octahydroindole-2-carboxylic acid are commercially available, However, the need remains of a synthesis free of the many drawbacks previously disclosed.

An object of the present invention is to develop an economical and efficient method for the preparation of Perindopril from the intermediate (2S,3aS,7aS)-octahydroindole-2-carboxylic acid or one of its aryl esters, without the need of purification resulting of the process disclosed in EP 0 308 341.

Another object of the present invention is to develop a favorable access to the intermediate (2S,3aS,7aS)-octahydroindole-2-carboxylic acid and/or to its aryl esters.

### Summary of the invention

The present invention provides a novel access to Perindopril with good yields and high purity, using as an intermediate the p-nitrobenzyl ester of (2S,3aS,7aS)-octahydroindole-2-carboxylic acid, of formula VI :

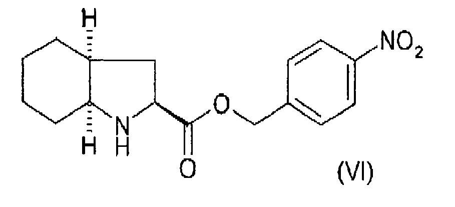

The coupling of the ester of formula VI with N-[(S)-1-ethoxycarbonyl-butyl]-(S)-alanine leads to a product which may be purified by crystallization, unlike its benzyl analog described in EP 0 308 341. After cleavage of the p-nitrobenzyl group, the purity level of the thus obtained Perindopril is far better than the level obtained according to EP 0 308 341.

The present invention also provides a favorable access to (2S,3aS,7aS)-octahydroindole-2-carboxylic acid and to its aryl esters, including the ester of formula VI. This access involves the asymmetric resolution of a salt of formula VII : 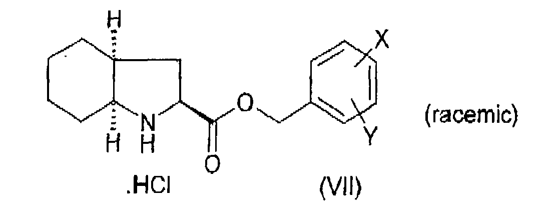 wherein X and Y are independently a hydrogen atom or a halogen, an alkyl, an alkoxyor a nitro- group,
with a chiral acid such as diaroyl-(L)-tartaric acid, (L)-mandelic acid, abietic acid or (D)-10-camphorsulphonic acid.

### Detailed description of the invention

An object of the present invention is to develop an economical and efficient method for the preparation of Perindopril from the intermediate (2S,3aS,7aS)-octahydroindole-2-carboxylic acid or one of its aryl esters, without the purification problems occurring in the process disclosed in EP 0 308 341.

According to EP 0 308 341, the synthesis of Perindopril involves the condensation of the (2S,3aS,7aS)-octahydroindole-2-carboxylic acid of formula VIII : 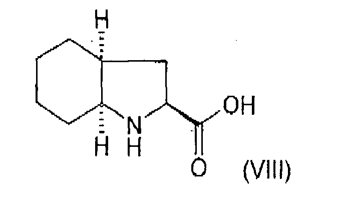 with benzyl alcohol in the presence of p-toluenesulphonic acid, to give the p-toluenesulphonic acid salt of the benzyl ester of formula IX :

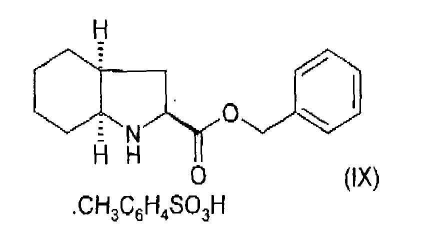

The product of formula IX is then coupled with the N-[(S)-1-ethoxycarbonyl-butyl]-(S)-alanine of formula X: 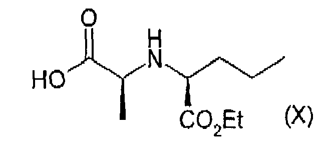 in alkaline conditions, in the presence of dicyclohexylcarbodiimide (DCC) and 1-hydroxy benzotriazole (HOBT), to give a coupling product of formula XI :

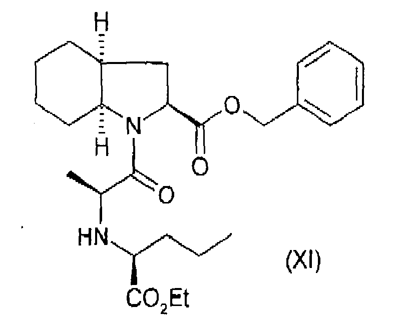

The benzyl ester of formula XI is then debenzylated by hydrogenolysis in the presence of palladium on charcoal. The product is solubilized in a mixture of ethyl acetate and isopropanol and tert-butylamine is added. The tert-butylamine salt of Perindopril of formula I is obtained by crystallization.

The coupling step, leading to the compound of formula XI, induces the formation of impurities of formulas II and II', due to the presence of coupling reagents such as DCC and HOBT. The coupling product of formula XI is obtained as an oil and cannot be further purified by crystallization to remove the impurities,

In order to avoid these inconveniences while maintaining the good yields provided by DCC and HOBT, it has been considered to modify the structure and reactivity of the starting products.

In WO 2004/099138, the formation of the benzene sulphonic acid salts of the esters of formula XII is described: 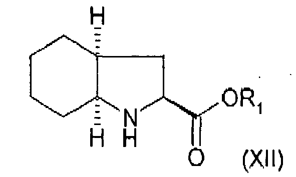 wherein R₁ is an optionally substituted aralkyl group, preferably selected from benzyl, 4-halo phenylmethyl and 4-C₁₋₄-alkoxy phenylmethyl, as intermediates for the synthesis of Perindopril.

As an example is described the coupling reaction of N-[(S)-1-ethoxycarbonyl-butyl]-(S)-alanine of formula X and the compound of formula XII, R₁ being 4-chloro phenylmethyl. The coupling reaction occurs in similar conditions as in EP 0 308 341. The yield is 78% and the product is obtained as an oil. The debenzylation of the 4-methoxyphenylmethyl analogue is also described.

Although a favorable coupling yield is obtained, this attempt to use substituted aralkyl esters of formula XII does not show obvious advantages towards the use of the corresponding benzyl ester. No new element is given about the purity of the coupling product. In the case of the 4-chloro phenylmethyl ester, the coupling product is obtained as an oil, without indication that it may crystallize to allow easier purification.

The only phenyl substituents mentioned in WO 2004/099138 are halogens and alkoxy-, which have electro- and mesomer- donor properties. It is difficult to anticipate what kind of result would give a substituent with very different physical or chemical properties.

In a surprising manner, the Applicant has found that the coupling of the p-nitrobenzylic ester of formula VI : 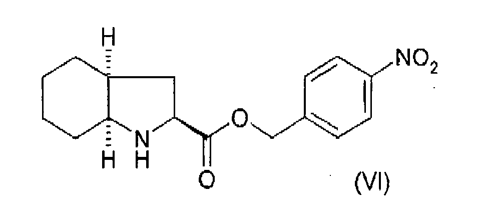 with the compound of formula XI, in the presence of DCC and HOBT, gives access to the product of formula XIII : 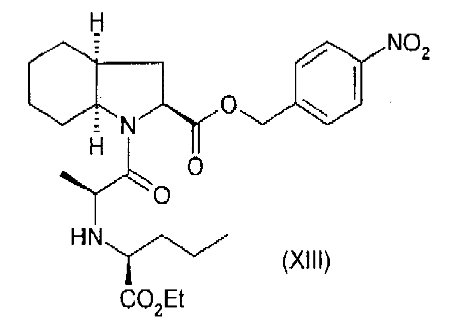 with excellent yield and purity, due to the fact that the compound of formula XIII may be purified by crystallization.

The crystallization properties of the coupling product of formula XIII may be attributed to the particular properties of the nitro group. Its electro- and mesomer- properties, being very distinct (acceptor) from those of the halogen and alkoxy substituents (donor), might explain a different result from WO 2004/099138. Accordingly, it is likely that products formed by the coupling of (2S,3aS,7aS)-octahydroindole-2-carboxylic acid p-nitrobenzylic ester with analogs of N-[(S)-1-ethoxycarbonyl-butyl]-(S)-alanine would also display crystallization properties. These properties would be useful for the synthesis of analogs of Perindopril.

A salt of the p-nitrobenzylic ester of formula VI may be obtained according to well-known methods, such as the coupling of (2S,3aS,7aS)-octahydroindole-2-carboxylic acid with p-nitrobenzyl alcohol, in the presence of a coupling agent such as an aryl sulphonic acid (benzene sulphonic acid, p-toluenesulphonic acid...). In a preferred embodiment, the reaction occurs in toluene and the forming water is removed by azeotropic distillation with a Dean-Stark apparatus.

The obtained salt, for example an aryl sulphonic salt, is then neutralized with a base such as triethylamine, leading to the free ester of formula VI : 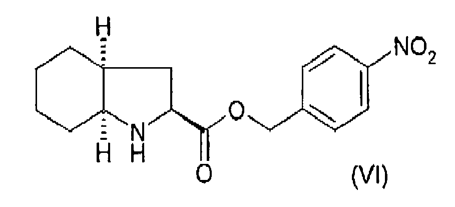 which is reacted with N-[(S)-1-ethoxycarbonyl-1-butyl]-(S)-alanine of formula X : 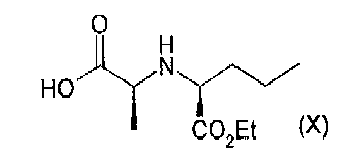 in the presence of dicyclohexylcarbodiimide (DCC) and 1-hydroxy benzotriazole (HOBT), to provide (2S,3aS,7aS)-1-{2-[1-ethoxycarbonyl-(S)-butylaminol-(S)-propionyl}-octahydroindole-2-carboxylic acid p-nitrobenzylic ester of formula XIII :

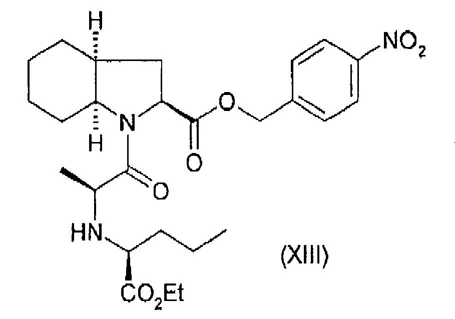

In a preferred embodiment, the solvent is a solvent of medium polarity, such as dichloromethane or ethyl acetate. In a preferred embodiment, the reaction temperature is close to ambient temperature, preferably from 15 to 35 °C. In a preferred embodiment, the amounts of HOBT and DCC are close to 1 equivalent, preferably comprised between 0.95 and 1.05 equivalents.

(2S,3aS,7aS)-1-2-{2-[1-ethoxycarbonyl-(S)-butylamino]-(S)-propionyl}-octahydroindole-2-carboxylic acid p-nitrobenzylic ester of formula XIII may be crystallized from a crystallization solvent such as an alkyl ether. A crystallization from diisopropyl ether led to a yield of 80% and a purity above 98% (HPLC).

The p-nitrobenzyl ester of formula XIII is then converted into the corresponding acid by hydrogenolysis in the presence of a catalyst. In a preferred embodiment, the conversion of the p-nitrobenzyl ester of formula XIII into the corresponding acid is achieved by hydrogenolysis using hydrogen in the presence of a catalyst selected from the group consisting of Pd/C, Rh/C, Raney nickel and Pt/C, and the like.

Perindopril of formula I is obtained : 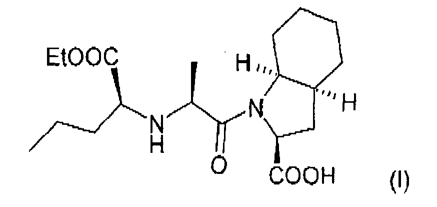 and may be converted into a salt, such as Perindopril erbumine of formula XIV: 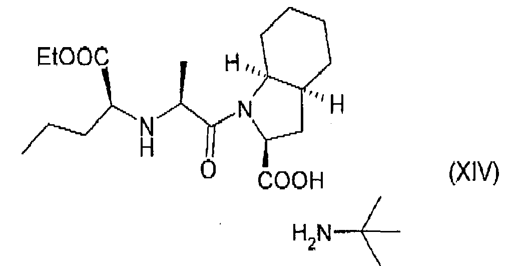 by reaction with the appropriate base, such as tert-butylamine. It may also be converted into the arginine salt by reaction with arginine.

According to this process, Perindopril erbumine is obtained with good yields and its purity is significantly better than according to the process described in EP 0 308 341. This improvement is due to the crystallization properties of the coupling product of formula XIII, which allow easier purification of this intermediate.

Another object of the present invention is to provide a favorable access to the intermediate compound (2S,3aS,7aS)-octahydroindole-2-carboxylic acid and/or to its aryl esters.

Different processes for the synthesis of (2S,3aS,7aS)-octahydroindole-2-carboxylic acid are disclosed in literature (see for example EP 1354875 or EP1354876). This compound is also commercially available.

in a preferred embodiment of the present invention, (2S,3aS,7as)-octahydroindole-2-carboxylic acid is synthesized in the following manner :

An ester of N-acetyl-β-acyloxy alanine of formula XV: 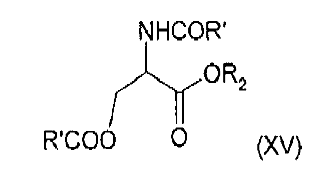 wherein R' is a lower alkyl radical such methyl, ethyl, propyl etc. and R₂ is a lower alkyl radical such as methyl, ethyl etc..., is reacted with an enamine of formula XVI : 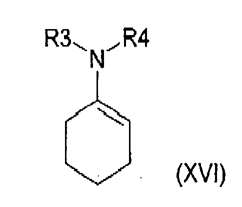 wherein R3 and R4 are each an alkyl group or together with nitrogen atom to which they are linked, a cyclic structure, which may contains an heteroatom selected from the group consisting of oxygen, sulphur or nitrogen, to give a cyclohexanone derivative of formula XVII: 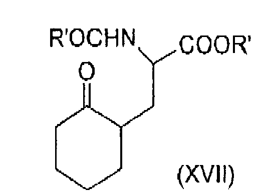 which is hydrolytically cyclized to give hexahydroindole-2-carboxylic acid of formula XVIII

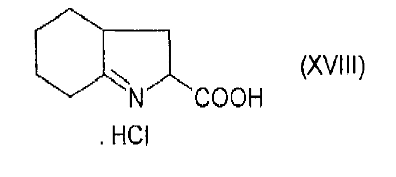

The imine of formula XVIII is then converted into the enamine of formula XVIII' : 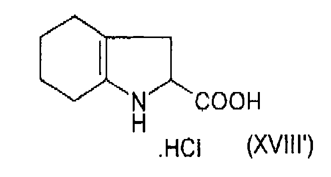 and then reduced into the racemate (R,R,R)/(S,S,S) of formula XIX : 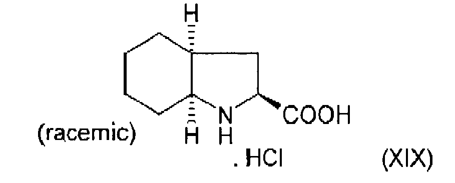 for example through hydrogenolysis, using hydrogen and a heterogeneous catalyst such as Pd/C, Rh/C, Pt/C, Raney nickel and the like.

The product of formula XIX is then esterified with an aryl alcohol of formula XX : 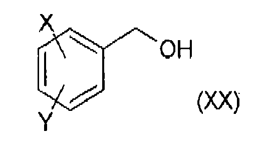 wherein X and Y are independently an hydrogen atom or a halogen, an alkyl, an alkoxyor a nitro- group, in the presence of an aryl sulphonic acid such as benzene sulphonic acid or p-toluenesulphonic acid.

The term "alkoxy-" means an oxygen atom attached to an alkyl group, such as methoxy-, ethoxy- and the like.

After treatment with HCl, a racemic salt of formula VII is obtained : 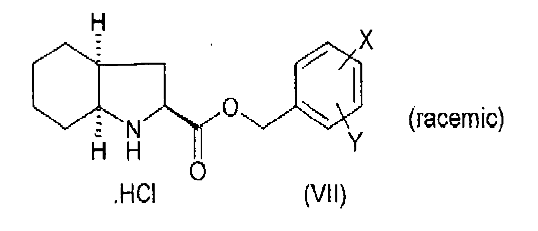 wherein X and Y are defined as above.

The Applicant observed that compound VII was easily resolved with chiral acids such as diaroyl-(L)-tartaric acid, (L)-mandelic acid, abietic acid or (D)-10-camphorsulphonic acid. It is a surprising result towards EP 115345 (p. 1, I. 22-26), where it is stated that the resolution of compounds such as octahydroindole-carboxylic esters with chiral acids give poor results. In the literature, the chiral compounds used for the asymmetric resolution of this family of products are chiral amines or amino-acids.

The term "diaroyl-(L)-tartaric acid" means (L)-tartaric acid substituted on both -OH by a carboxy (C=O) group attached to an aryl group. Examples of commercially available compounds are dibenzoyl-(L)-tartaric acid or di-p-toluoyl-(L)-tartaric acid.

The resolution may occur in a solvent such as alkanes or alkyl ethers, for example diisopropyl ether.

An enantiomerically pure salt of formula XXI is then obtained : 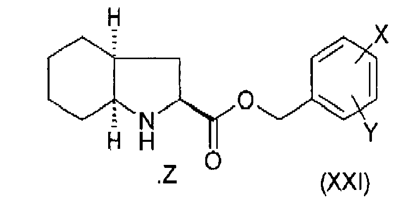 where Z is an optically active carboxylic acid such as diaroyl-(L)-tartaric acid, (L)-mandelic acid, abietic acid or (D)-10-camphorsulphonic acid.

After the cleavage of the aryl ester, for example by catalytic hydrogenation, and neutralization, (2S,3aS,7aS)-octahydroindole-2-carboxylic acid is obtained. It may be used as a starting product in the process described above, for the synthesis of Perindopril.

In a particular embodiment of the invention, the compound of formula XX is p-nitrobenzyl alcohol. The resulting enantiomerically pure salt of formula XXIa : 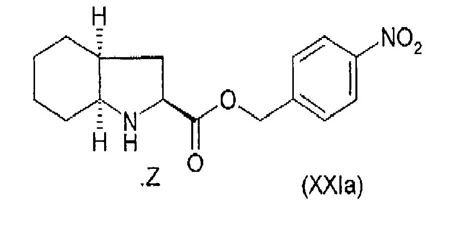 where Z is an optically active carboxylic acid such as diaroyl-(L)-tartaric acid, (L)-mandelic acid, abietic acid or (D)-10-camphorsulphonic acid,
may be used, after neutralization, in a coupling reaction with N-[(S)-1-ethoxycarbonylbutyl]-(S)-alanine of formula X according to the process described above, to give access to the compound of formula XIII, which is further converted into Perindopril or one of its salts.

### Conclusion

The present invention provides a process for the synthesis of Perindopril with good yields and excellent purity, as well as results unexpected from the literature in describing the asymmetric resolution of an aryl octahydroindole-2-carboxylate with chiral acids.

The following examples illustrate the present invention in more details, but are not to be considered to limit its scope in any manner. Melting points are uncorrected.

### Examples :

### Example 1

### Preparation of octahydroindole-2-carboxylic acid hydrochloride

10 Kg of hexahydroindole-2-carboxylic acid hydrochloride were charged into 50 L of glacial acetic acid in a hydrogenator kettle. 0.5 Kg of platinum 10% on charcoal was charged and temperature was raised to 60-70°C. Hydrogen was supplied till the consumption ceased. The catalyst was filtered and the filtrate concentrated under reduced pressure. After complete removal of the solvent, 10 L of n-butanol were added, the mixture was cooled to 20-25°C and stirred for 30 min. The precipitated crystals were centrifuged and dried. The amount of product obtained was 8.2 Kg.
NMR ¹H : 1.28 (m, 2H ; -CH₂); 1.68 (m, 2H ; -CH₂); 1.72 (m, 2H ; -CH₂); 2.02 (m, 2H ; - CH₂) 2.21 (m, 1 H ; -CH); 2.41 (m, 2H ; -CH₂); 3.04 (m,1 H ; -CH); 3.91 (m, 1 H ; -CH)

### Example 2

### Preparation of phenylmethyl octahydroindole-2-carboxylate hydrochloride

8.0 Kg of octahydroindole-2-carboxylic acid hydrochloride, 35 L of toluene and 6.5 Kg of benzyl alcohol were charged into a 200 L glass-lined reactor. 8 L of p-toluene sulfonic acid were added and water was removed azeotropically. After the required quantity of water was collected, the temperature was lowered to 25-30°C, 25 L of water were added and pH was adjusted to 10,5, The organic layer was separated and the aqueous layer discarded. The organic layer was distilled off completely. After the distillation, 25 L of methanol and 1.8 Kg of 10% methanolic hydrochloric acid were added. The mass was cooled to 10-15°C and centrifuged. 11.0 Kg of the product were obtained, with a melting point of 143-145°C.
NMR ¹H : 2.0 (NH); 3.58 (-CH); 1.9 (-CH₂); 1.66 (-CH); 1.40 (-CH₂); 1.44 (2 X-CH₂) ; 1.52 (-CH₂); 2.56 (CH); 5.34 (-CH₂); 7.19 (5 H aromatic protons)

### Example 3

### Preparation of (2S,3aS,7aS)-phenylmethyl-octahydroindole-2-carboxylate hydrochloride

10 Kg of phenylmethyl octahydroindole-2-carboxylate hydrochloride and 40 L of water were charged into a 100 L glass reactor. The pH was adjusted to 10.5 using aqueous sodium hydroxide. 50 L of diisopropylether were added and the organic layer was separated. The organic phase was dried. 3.5 Kg of dibenzoyl-(L)-tartaric acid were added to the organic layer and stirred for one hour at room temperature. The crystallized salt was centrifuged and the wet cake was taken in another reactor, which contained 30 L of water. The pH was adjusted to 9.5 with sodium hydroxide and 25 L of dichloromethane were added. The organic layer was separated and dried on magnesium sulphate. The organic layer was distilled off and after complete removal of the solvent, 2 Kg of 10% methanolic hydrochloric acid and 20 L of methanol were added to the residue. The crystallized solid was stirred at 0-5°C for one hour. The reaction mass was centrifuged and dried to get 4.7 Kg of (2S,3aS,7as) phenylmethyl octahydroindole-2-carboxylate hydrochloride, with an optical rotation of -32 (ethyl acetate).

### Example 4

### Preparation of (2S,3aS,7a5)-octahydroindole-2-carboxylic acid p-nitrobenzylic ester p-toluenesulfonate

In a 1 L flask with mechanical stirring and equipped with a Dean-Stark apparatus were mixed 40 g of (2S,3aS,7aS)-octahydroindole-2-carboxylic acid (0.236 mmole), 40 g of p-nitrobenzyl alcohol (~1.1 mol. eq.), 54 g of p-toluenesulfonic acid (1.2 mol. eq.) and 400 mL of toluene.
The reaction was heated under reflux for 3 hours while water was removed by azeotropic distillation with a Dean-Stark apparatus.
The reaction was cooled to room temperature then 400 ml of isopropanol were added and the solution was stirred at 0-5°C for 1 hour.
The solid was removed by filtration, rinsed with isopropanol and dried at 50°C in *vacuo* for one night to give 89 g of ester (79%).
NMR-¹H : 8.28 (d, J = 8.6, 2H arom. APTS), 7.71 (d, J = 8, 2H arom. Bn), 7.68 (c, J = 8.6, 2H arom. APTS), 7.24 (d, J = 8, 2H arom. Bn), 5.44 (m, CH₂ Bn), 4.59 (bt, J = 8.4, H), 3.73 (m, 1H), 2.53-2.45 (m, 2H), 2.37 (s, CH₃ APTS), 2.28-2.17 (m, 1 H), 1.98-1.90 (m, 1 H), 1.76-1.36 (m, 7H).

### Example 5

### Preparation of (2S,3aS,7aS)-1-{2-[1-ethoxycarbonyl-(S)-butylamino]-(S)-propionyl}-octahydroindole-2-carboxylic acid p-nitrobenzylic ester

Solution A : to 1.12 L of dichloromethane at room temperature were added 34.4 g of N-[(S)-1-ethoxycarbonyl-1-butyl]-(S)-alanine under stirring.
Solution B : to a stirred solution of 75.4 g of (2S,3aS,7aS)-octahydroindole-2-carboxylic acid p-nitrobenzyl ester p-toluenesulfonate (1.0 eq. mol.) in 0.5 L of dichloromethane were added 16 g of triethylamine (1.0 eq. mol.).
After 10 minutes at room temperature, solution B was added to solution A, then 21.4 g of HOBT (1.0 eq. mol.) were added and the mixture was stirred for 15 minutes at room temperature.
The reaction was cooled to 0-5°C then 32.6 g of N,N-dicyclohexylcarbodiimide (0.95 eq.mol.) were added. The mixture was stirred for 5 h at room temperature.
The reaction mixture was cooled to 0-5°C for 1 hour then the solid was removed by filtration and rinsed with 2 x 40 mL of dichloromethane,
The filtrate was washed successively with : 0.6 L of NaHCO₃ 5% in water, 0.6 L of brine and 0.6 L of water.
The organic solvent was removed *in vacuo* at 40°C to give 86 g of an oily product.
30 ml of diisopropylether was added to the oil and the mixture was stirred 30 minutes at 0-5°C. the solid was removed by filtration and rinsed with 40 mL of diisopropylether.
154 mL of diisopropylether and 377 mL of n-heptane are added to the solution.
The mixture was heated at 50°C then cooled gently to room temperature.
The mixture was stirred for 2 hours at 0-5°C. The crystalline product was filtered and rinsed with heptane.
The product was dried at 50°C *in vacuo* for 16 h to give 63 g (80%) of (2S,3aS,7aS)-1-{2-[1-ethoxycarbonyl-(S)-butylamino]-(S)-propionyl}-octahydroindole-2-carboxylic acid p-nitrobenzylic ester with a very high purity (>98% HPLC) and a melting point of 68-72°C.
NMR-¹H : 8.22 (m, 2H arom.), 7.52 (m, 2H arom.), 5.27 (AB, CH₂Ph), 4.56 (dd, J = 10, 8.4, 1H), 4.30 (dq, J = 6.0-9.5-1.8, 2H), 4.6-4.1 (m, 2H), 3.85 (m, 1H), 2.62 (m, 1H), 2.26-1.94 (m, 5H), 1.75 (m, 4H), 1.51-1.40 (m, 2H), 1.37-1.20 (m, 3H), 1.32 (t, CH₃, H), 0.98 (t, CH₃, H).
MS (IC-NH₃) : 505 (M+1, 19), 504 (M, 68), 173 (10), 172 (99), 155 (20), 144 (14), 124 (17), 123 (11), 122 (99).

### Example 6

### Preparation of (2S,3aS,7aS)-1-{2-[1-ethoxycarbonyl-(S)-butylamino]-(S)-propionyl}-octahydroindole-2-carboxylic acid (Perindopril)

60 g of the above p-nitro ester were solubilized in 0.3 L of isopropanol under nitrogen, then 2 g of Pd/C were added and the solution was stirred under hydrogen (1 atm) for 3 h at room temperature.
The mixture was filtered, rinsed with isopropanol and concentrated *in vacuo* to give an oily product.

### Example 7

### Preparation of t-butylamine-(2S,3aS,7aS)-1-{2-[1-ethoxycarbonyl-(S)-butylamino]-(S)-propionyl}-octahydroindole-2-carboxylate) (Perindopril erbumine)

The above oily product was solubilized in a mixture of 27 ml of isopropanol and 266 mL of ethyl acetate.
10.5 mL of t-butylamine were added, the solution was heated to 30-35°C and cooled to room temperature and then to 0-5°C.
The product was isolated by filtration, rinsed with ethyl acetate and dried at 38°C *in vacuo* for 16 h to give 23.5 g (90% yield) of the desired product.

The same procedure could be applied to the production of the arginine salt of Perindopril.

## Claims

1. (2S,3aS,7aS)-octahydroindole-2-carboxylic acid p-nitrobenzylic ester of formula VI : 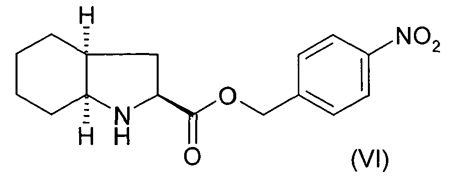 as an intermediate for the synthesis of Perindopril.

2. (2S,3aS,7aS)-1-{2-[1-ethoxycarbonyl-(S)-butylamino]-(S)-propionyl}-octahydroindole-2-carboxylic acid p-nitrobenzylic ester of formula XIII : 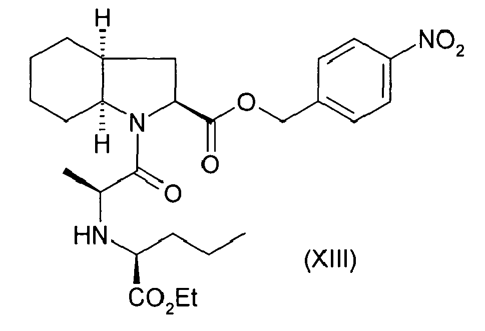 as an intermediate for the synthesis of Perindopril.

3. A process for the synthesis of Perindopril with high purity, which comprises the steps of :
a) Reacting (2S,3aS,7aS)-octahydroindole-2-carboxylic acid p-nitrobenzylic ester with N-[(S)-1-ethoxycarbonyl-1-butyl]-(S)-alanine in the presence of dicyclohexylcarbodiimide and 1-hydroxy benzotriazole, to give (2S,3aS,7aS)-1-{2-[1-ethoxycarbonyl-(S)-butylamino]-(S)-propionyl}-octahydroindole-2-carboxylic acid p-nitrobenzylic ester of formula XIII : 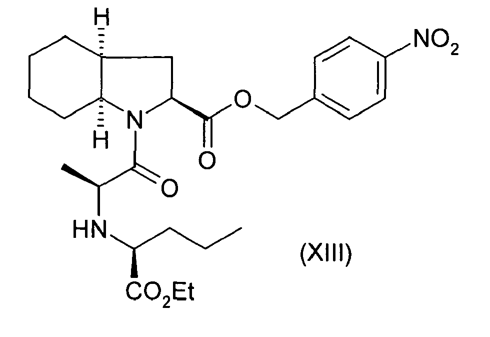
b) purifying the compound of formula XIII by crystallization ;
c) converting the purified p-nitrobenzyl ester of formula XIII into the corresponding acid by hydrogenolysis, to yield Perindopril of formula I :

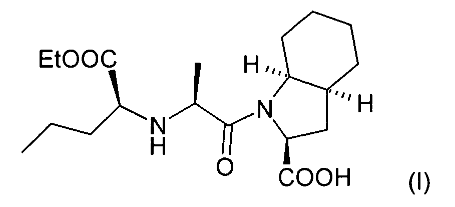

4. A process as claimed in claim 3, wherein (2S,3aS,7aS)-octahydroindole-2-carboxylic acid p-nitrobenzylic ester, in the form of a salt, is neutralized with a base before being reacted with N-[(S)-1-ethoxycarbonyl-1-butyl]-(S)-alanine.

5. A process as claimed in claim 3 or claim 4, wherein the coupling reaction between (2S,3aS,7aS)-octahydroindole-2-carboxylic acid p-nitrobenzylic ester and N-[(S)-1-ethoxycarbonyl-1-butyl]-(S)-alanine occurs in a solvent of medium polarity, such as dichloromethane or ethyl acetate.

6. A process as claimed in one of the claims 3 to 5, wherein the amounts of dicyclohexylcarbodiimide and 1-hydroxy benzotriazole are comprised between 0.95 and 1.05 equivalents.

7. A process as claimed in one of the claims 3 to 6, wherein the compound of formula XIII is purified by crystallization from a solvent of low polarity, such as an alkyl ether, an alkane or a mixture thereof.

8. A process as claimed in one of the claims 3 to 7, wherein the conversion of the p-nitrobenzyl ester of formula XIII into the corresponding acid is achieved by hydrogenolysis, using hydrogen in the presence of a catalyst selected from the group consisting of Pd/C, Rh/C, Raney nickel, Pt/C, and the like.

9. A process as claimed in one of the claims 3 to 8, wherein the (2S,3aS,7aS)-octahydroindole-2-carboxylic acid p-nitrobenzylic ester is prepared by reacting (2S,3aS,7aS)-octahydroindole-2-carboxylic acid and p-nitrobenzyl alcohol in the presence of an aryl sulphonic acid.

10. A process for the synthesis of (2S,3aS,7aS)-octahydroindole-2-carboxylic acid, which comprises the steps of :
a) esterifying the (R,R,R)/(S,S,S) octahydroindole-2-carboxylic acid hydrochloride of formula XIX: 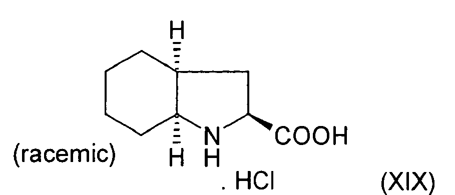 with an aryl alcohol of formula XX : 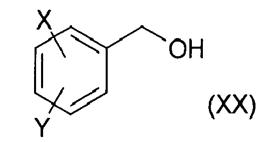 wherein X and Y are independently a hydrogen atom or a halogen, an alkyl, an alkoxy- or a nitro- group, in the presence of an aryl sulphonic acid, to form an ester of formula VII: 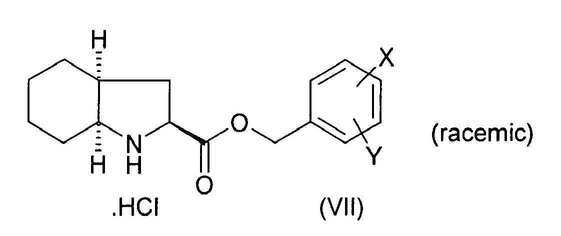
b) resolving the ester of formula VII using an optically active organic acid selected among diaroyl-(L)-tartaric acid, (L)-mandelic acid, abietic acid and (D)-10-camphorsulphonic acid ;
c) converting the enantiomerically pure ester of formula XXI : 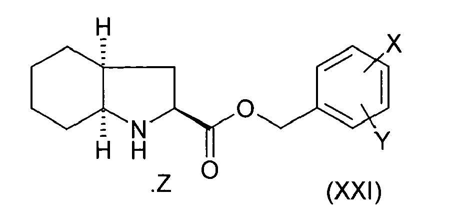
where Z is an optically active carboxylic acid such as diaroyl-(L)-tartaric acid, (L)-mandelic acid, abietic acid or (D)-10-camphorsulphonic acid,
into the corresponding acid by hydrogenolysis, using hydrogen and a catalyst selected among Pd/C, Rh/C, Pt/C, Raney nickel and the like.

11. A process for the synthesis of (2S,3aS,7aS)-octahydroindole-2-carboxylic acid p-nitrobenzylic ester, which comprises the steps of:
a) esterifying the (R,R,R)/(S,S,S) octahydroindole-2-carboxylic acid of formula XIX with p-nitrobenzyl alcohol in the presence of an aryl sulphonic acid, to form the racemate of the ester of formula VI : 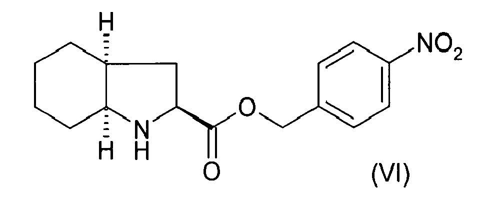
b) resolving the ester of formula VI using an optically active organic acid selected among diaroyl-(L)-tartaric acid, (L)-mandelic acid, abietic acid and (D)-10-camphorsulphonic acid ;
c) neutralizing the resulting salt with a base.
